(19) Europäisches Patentamt

European Patent Office

Office européen des brevets

(11) **EP 0 560 331 B1**

(12) **EUROPÄISCHE PATENTSCHRIFT**

(45) Veröffentlichungstag und Bekanntmachung des Hinweises auf die Patenterteilung:
**17.02.1999 Patentblatt 1999/07**

(51) Int. Cl.[6]: **A61B 6/04**, G05D 3/20, G05B 19/18

(21) Anmeldenummer: 93103855.8

(22) Anmeldetag: **10.03.1993**

(54) **Vorrichtung und Verfahren zur Positionierung eines Körperteils für Behandlungszwecke**

Device and method for positioning a part of the body for therapeutic treatment

Dispositif et procédé de positionnement d'une partie du corps pour le traitement thérapeutique

(84) Benannte Vertragsstaaten:
AT BE CH DE DK ES FR GB GR IE IT LI LU MC NL PT SE

(30) Priorität: **11.03.1992 DE 4207632**

(43) Veröffentlichungstag der Anmeldung:
**15.09.1993 Patentblatt 1993/37**

(73) Patentinhaber:
**Deutsches Krebsforschungszentrum**
**69120 Heidelberg (DE)**

(72) Erfinder:
• **Sturm, Volker, Prof. Dr. med.**
**W-6908 Wiesloch-Schatthausen (DE)**
• **Pastyr, Otto**
**W-6906 Leimen-St. Ilgen (DE)**
• **Schlegel, Wolfgang, Dr. rer. nat.**
**W-6900 Heidelberg (DE)**
• **Uihlein, Christoph, Dr.**
**W-7770 Überlingen (DE)**
• **Mack, Thomas, Dr.**
**W-7771 Altheim (DE)**

(74) Vertreter:
**Castell, Klaus, Dr.-Ing. et al**
**Patentanwaltskanzlei**
**Liermann - Castell**
**Schillingsstrasse 335**
**52355 Düren (DE)**

(56) Entgegenhaltungen:
EP-A- 0 114 505          EP-A- 0 480 035
US-A- 3 861 807          US-A- 4 146 924
US-A- 4 396 945          US-A- 4 705 955

**Beschreibung**

Die Erfindung betrifft eine Vorrichtung zur Positionierung eines Körperteils zur Behandlung mit einem medizinischen Gerät , enthaltend

(a) Mittel zum Erzeugen von optischen Informationen der Position des Körperteils relativ zu dem medizinischen Gerät,

(b) mindestens zwei bildsignalerzeugende Sensoren, welche zur Beobachtung der optischen Informationen ausgerichtet sind,

(c) bildverarbeitende Mittel zur Verarbeitung der aus den optischen Informationen erzeugten Bildsignale der Sensoren,

(d) Signalverarbeitungs- und Reglermittel

-    zum Ermitteln der Istposition des Körperteils aus der beobachteten optischen Informationen,

-    zum Vergleich der Istposition mit einer Sollposition und

-    zur Erzeugung von Stellsignalen, die von der Regelabweichung zwischen Istposition und Sollposition abhängen, und

(e) Mittel, die von den Stellsignalen beaufschlagt sind, zur Positionierung des Körperteils, so daß ein genau definierter Behandlungspunkt in einer Sollposition gehalten wird, und zur Verhinderung einer Behandlung bei Fehlausrichtung zwischen Gerät und Körperteil.

Eine solche Vorrichtung ist durch die US-A-3,861,807 bekannt. Bei dieser Vorrichtung wird ein kleiner Reflektor an der Haut des zu behandelnden Körperteils angebracht. Eine Lichtquelle wird auf das Körperteil gerichtet. Die Lichtquelle erzeugt konzentrische Ringe mit unterschiedlicher Intensität. Die Intensität des von dem Reflektor reflektierten Lichts wird durch eine fotoelektrische Zelle gemessen. Wenn sich der Reflektor in der Mitte des Lichtstrahls befindet, ist die Intensität am größten. Der zu behandelnde Körperteil wird in Abhängigkeit von der durch die fotoelektrische Zelle gemessene Intensität so bewegt, daß die zu behandelnde Stelle bzgl. des medizinischen Geräts sich immer am gleichen Ort befindet. Dadurch wird der Körperteil so positioniert, daß ein genau definierter Behandlungspunkt in einer Sollposition gehalten wird. Es können auch Lichtquellen und Reflektoren paarweise vorhanden sein.

Insbesondere bezieht sich die Erfindung auf die Bestrahlung von Tumoren im Gehirn mittels eines Bestrahlungsgerätes. Dabei sollte der Strahl des Bestrahlungsgerätes auf Bruchteile eines Millimeters genau auf den Tumor treffen. Dadurch wird einer Beschädigung gesunden Gewebes entgegengewirkt und die Strahlung optimal ausgenutzt. Es hat sich als vorteilhaft erwiesen, bei einer solchen Bestrahlung nicht die gesamte Dosis auf einmal zur Einwirkung zu bringen. Vielmehr sollten eine Anzahl von kleineren Dosen in zeitlichen Abständen aufgebracht werden. Weiterhin sollten die Strahlendosen vorteilhafterweise aus verschiedenen Richtungen eingestrahlt werden. Dadurch verteilt sich die Strahlung, die auch auf das umliegende, gesunde Gewebe trifft, auf einen größeren Bereich. Die Gefahr der irreparablen Schädigung von gesundem Gewebe wird daher vermindert. Die Strahlen, die von dem Bestrahlungsgerät ausgehen, müssen sich bei der Aufbringung der verschiedenen Dosen genau in einem Isozentrum im Bereich des zu bestrahlenden Tumors schneiden. Das setzt aber voraus, daß der Kopf des Patienten mit entsprechender Genauigkeit reproduzierbar positioniert und ausgerichtet wird.

In der Praxis wird dazu ein stereotaktisches Verfahren angewandt: An dem Kopf des Patienten wird ein Metallring durch Festschrauben am Schädelknochen befestigt. Mit dem Metallring ist ein Acrylglaszylinder verbunden. In den Acrylglaszylinder sind Metalldrähte eingegossen, die ein kopffestes Koordinatensystem definieren. Vom Kopf des Patienten mit diesem Ring und dem Acrylglaszylinder und Metalldrähten wird z.B. ein Computertomogramm aufgenommen. In dem Computertomogramm sind die Metalldrähte sichtbar. Die Lage des ebenfalls in dem Computertomogramm erscheinenden Tumors kann dann in bezug auf ein durch die Metalldrähte definiertes Koordinatensystem auf Bruchteile eines Millimeters genau bestimmt werden.

Der Tumor (Behandlungspunkt), dessen Lage so bestimmt wurde, wird dann durch ein optisches Ausrichtsystem genau zu dem Isozentrum ausgerichtet, durch das der Strahl des Bestrahlungsgerätes stets hindurchgeht.

Die EP-A-0 114 505 offenbart ein Verfahren oder eine Vorrichtung zur Kalibrierung eines Roboters, wobei die Kalibriermittel Einrichtungen zur Ermittlung der Position eines Roboterarmes enthalten, wenn der Arm eine Arbeitsstation erreicht. Zu diesem Zweck ist eine Zielscheibe mit mehreren Markierungen an dem Roboterarm angebracht. Die.

Zielscheibe wird durch nicht mit dem Roboterarm verbundenen Kameras aus unterschiedlichen Winkeln beobachtet. In einem Rechner wird hieraus die Istposition des Roboterarmes berechnet. Der Istposition wird mit der Sollposition verglichen und eine Kalibrierung durchführt.

Die US-A-4,705,955 bildet einen technologischen Hintergrund. Dort ist ein Tisch gezeigt, auf dem ein Patient liegen soll, der bestrahlt wird und wobei kleine Bewegungen des Patienten in bezug auf den Tisch während der tatsächlichen Bestrahlung festgestellt werden sollen und die Position des Tisches dementsprechend berichtigt werden soll.

Die US-A-4,396,945 zeigt eine Vorrichtung, bei der die Lokation eines Elementes im Raum, beispielsweise eines Roboterarms oder einer anderen Plattform, genau durch elektrooptische Mittel festgestellt werden kann.

Die US-A-4,146,924 zeigt eine ähnliche Anordnung.

Die bekannten Verfahren zur Vermessung und Festlegung des Kopfes des Patienten sind zeitaufwendig und daher nicht für mehrfache Anwendung geeignet.

Der Erfindung liegt die Aufgabe zugrunde, einen Körperteil eines Patienten zur Behandlungszwecken in einer wohldefinierten und reproduzierbaren Position relativ zu einem Gerät zu halten.

Insbesondere liegt der Erfindung die Aufgabe zugrunde, den Kopf des Patienten bei einer fraktionierten Bestrahlung mit einem Bestrahlungsgerät bei aufeinanderfolgenden Behandlungen in einer genau definierten Position relativ zu dem Strahl des Bestrahlungsgerätes zu halten.

Erfindungsgemäß wird diese Aufgabe dadurch gelöst, daß

(f) die Mittel zum Erzeugen von optischen Informationen der Position des Körperteils relativ zu dem medizinischen Gerät mindestens zwei Markierungen aufweisen, welche eingerichtet sind, in genau definierten Positionen an dem Körperteil angebracht zu werden,

(g) die bildsignalerzeugenden Sensoren Mittel zur Erzeugung einer Pixelmatrix enthalten, in welcher die Markierungen als flächige Gebilde erscheinen,

(h) die bildverarbeitenden Mittel zur Bestimmung der Schwerpunkte der so erhaltenen flächigen Gebilde als Markierungspunkte ausgelegt sind, die durch die Markierungen bestimmt sind und die Istposition des Körperteils definieren, und

(i) die Signalverarbeitungs- und Reglermittel so zusammenwirken, daß die Ermittlung der Istposition des Körperteils aus der beobachteten optischen Informationen durch Berechnung erfolgt.

Nach der Erfindung wird die Position des Kopfes oder sonstigen Körperteils durch Abtastung der Markierung mittels der Sensoren bestimmt. Durch einen Regelkreis wird die richtige Position aufrechterhalten. Es hat sich überraschenderweise gezeigt, daß auf diese Weise eine höhere Genauigkeit der Positionierung erzielbar ist als durch mechanische Fixierung. Statt einer Nachführung des Kopfes zur Ausrichtung des Behandlungspunktes nach dem Isozentrum kann auch eine Ablenkung des Strahls erfolgen. Im Notfall kann bei einer Fehlausrichtung das Behandlungs gerät abgeschaltet werden.

Der Sensor enthält Mittel zur Erzeugung einer Pixelmatrix, in welcher die Markierungen als flächige Gebilde erscheinen. Die bildverarbeitenden Mittel sind zur Bestimmung der Schwerpunkte der so erhaltenen flächigen Gebilde als Markierungspunkte ausgelegt. Das Auflösungsvermögen üblicher Sensoren dieser Art ist geringer als die geforderte Positioniergenauigkeit. Wenn man aber den Schwerpunkt eines von diesen Sensoren erfaßten flächigen Gebildes mit üblichen Mitteln der Mustererkennung bildet, also gewissermaßen über das relativ grobe Raster mittelt, dann ist dieser Schwerpunkt mit der erforderlichen Genauigkeit definiert. Es können so durch die Abtastung der relativ ausgedehnten Markierungen sub-pixelgenaue "Markierungspunkte" bestimmt werden. Diese legen die Istposition des zu behandelnden Körper teils mit hinreichender Genauigkeit fest.

Die Markierungen können von Kugeln gebildet sein, die in der Pixelmatrix unabhängig von der Beobachtungsrichtung als kreisförmige Gebilde erscheinen. Zur Vereinfachung der Bildverarbeitung ist es dabei zweckmäßig, wenn die Kugeln von kontrastierenden Flächen hinterlegt sind. Es kann eine Beleuchtungseinrichtung zur Beleuchtung der Kugeln von mehreren Seiten vorgesehen sein.

Zur genauen Positionierung des Kopfes eines Patienten relativ zu einer Bestrahlungseinrichtung, wobei der Behandlungspunkt in einem zu bestrahlenden Tumor liegt, enthält die Vorrichtung einen Bestrahlungstisch und einen auf dem Bestrahlungstisch in waagerechter Längsrichtung verschiebbaren Schlitten, auf welchem der Patient bei Benutzung der Vorrichtung liegt, und eine von dem Bestrahlungstisch gesonderte Kopfauflage, auf welcher bei Benutzung der Vorrichtung der Kopf des Patienten feststellbar aufliegt. Ein erstes und ein zweites der besagten Stellglieder greifen in vertikaler Richtung und in waagerechter Querrichtung an der Kopfauflage an zur Bewegung derselben relativ zu dem Bestrahlungstisch. Ein drittes Stellglied greift in der waagerechten Längsrichtung an dem Schlitten an zur Längsverschiebung des Schlittens.

Auf diese Weise wird der Kopf des Patienten auf und ab und seitlich bewegt. Es erfolgt aber keine Streckung in Längsrichtung.

Ein Ausführungsbeispiel der Erfindung ist nachstehend unter Bezugnahme auf die zugehörigen Zeichnungen näher erläutert.

Fig.1     ist eine schematisch-perspektivische Darstellung einer Bestrahlungsvorrichtung zur Bestrahlung eines Gehirntumors mittels eines Linearbeschleunigers, wobei die Strahlendosis fraktioniert aus verschiedenen Richtungen zur Einwirkung gebracht wird.

Fig.2     veranschaulicht die verschiedenen Richtungen des Strahls, wobei der Strahl jedesmal durch ein in dem Tumor liegendes Isozentrum geht.

Fig.3     zeigt schematisch eine Anordnung mit zwei bildsignalerzeugenden Sensoren (Kameras), durch welche mittels am Patienten anzubringender Markierungen eine definierte, einmal genau vermessene Lage des Patienten zu der Bestrahlungsvorrichtung wiederherstellbar ist, derart, daß der Strahl der Bestrahlungsvorrichtung bei späteren Bestrahlungen wieder genau den Tumor trifft.

Fig.4     ist ein Diagramm das schematisch die einzelnen Schritte der Bild- und Signalverarbeitung bei der Anordnung von Fig.3 veranschaulicht.

Fig.5     ist eine schematische Darstellung der von den Sensoren gelieferten Pixelmatrix und veranschaulicht die Bildverarbeitung.

Fig.6     ist eine schematische Darstellung eines Sonsors im Längsschnitt.

Fig.7     veranschaulicht in übertriebener Darstellung die Charakteristik der bei der Abbildung in dem Sensor auftretenden Abbildungsfehler.

Fig.8     veranschaulicht die Bestimmung der Lage der Sensoren anhand eines Referenzkörpers.

Fig.9     zeigt die am Kopf des Patienten angebrachten Markierungen.

Fig.10    zeigt die Stellglieder, durch welche der Kopf des Patienten in eine gewünschte Soll-Lage gebracht wird.

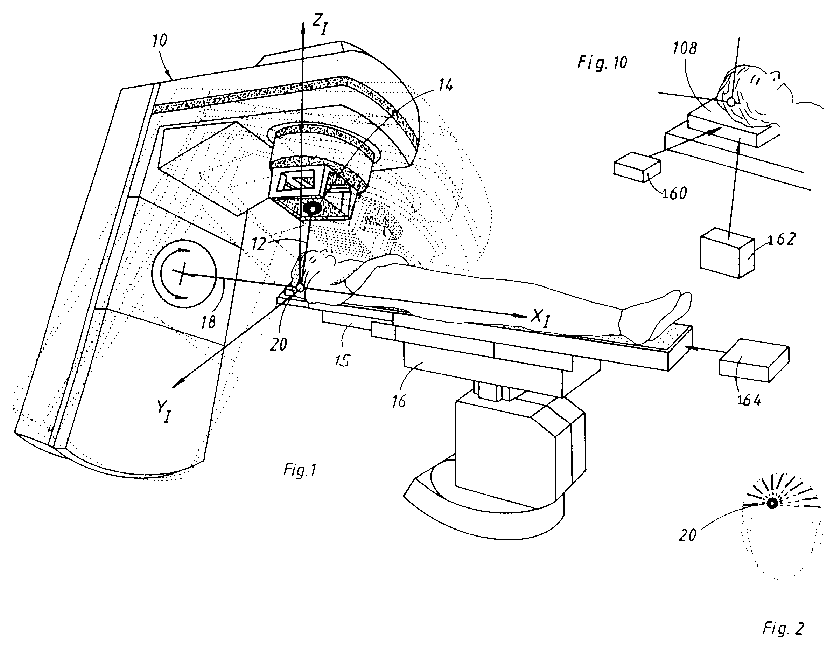

In Fig.1 ist schematisch eine Bestrahlungsvorrichtung zum Bestrahlen eines Gehirntumors dargestellt. Die Bestrahlungsvorrichtung ist mit 10 bezeichnet. Sie erzeugt einen Strahl 12 hochenergetischer Gamma-Sekundärstrahlung. Der Querschnitt des Strahls 12 ist durch eine Blende 14 bestimmt. Ein Patient liegt auf einem Bestrahlungstisch 16.

Um einen Tumor nacheinander aus verschiedenen Richtungen bestrahlen zu können und damit die Belastung des vom Strahl durchsetzten gesunden Gewebes gering zu halten, ist die Bestrahlungsvorrichtung 10 um eine Achse 18 verschwenkbar. Der Strahl 12 schneidet die Achse 18 in einem Isozentrum 20. Der Tumor muß durch geeignete Einstellung des Bestrahlungstisches in dieses Isozentrum gebracht werden. Wenn das der Fall ist, dann geht der Strahl auch bei einer Schwenkung der Bestrahlungsvorrichtung 10 um die Achse 18 stets durch den Tumor, wie in Fig.2 angedeutet ist.

Zunächst wird die genaue Position des zu bestrahlenden Tumors im Schädel des Patienten bestimmt. Das ist eine bekannte und daher hier nicht dargestellte Technik. Dabei wird am Schädel des Patienten ein Ring befestigt. An dem Ring sind Acrylglaskörper mit Metalldrähten angebracht. Die Metalldrähte dienen als Markierungen. Von dem Kopf des Patienten mit diesem Ring wird ein Computertomogramm oder ein Kernspin-Tomogramm erstellt, welches die Lage des Tumors und der Metalldrähte erkennen läßt. Die Lage des Tumors ist dann in einem durch die Metalldrähte definierten Koordinatensystem gegeben.

Bei der ersten Bestrahlung wird der zu bestrahlende Körperteil mittels stereotaktischer Verfahren genau ausgerichtet und fixiert. Dann befindet sich der Behandlungspunkt, also etwa ein Tumor, genau im Isozentrum des Gerätes. Das Isozentrum ist ein definierter Punkt in dem gerätefesten Koordinatensystem.

Es gilt nun, bei späteren Bestrahlungen den Körperteil wieder genau in diese einmal vermessene Lage zu bringen. Das sollte ohne die geschilderten stereotaktischen Hilfsmittel und in wesentlich kürzerer Zeit geschehen.

Zu diesem Zweck werden an dem Patienten vor der ersten Vermessung Markierungen angebracht, die eine reproduzierbare Position in bezug auf den Schädel des Patienten haben. Das sind bei dem beschriebenen Ausführungsbeispiel Dübel, die in den Knochen des Schädels eingelassen sind.

Diese Ausführungsart fällt nicht unter die Definition des in Anspruch 10 beanspruchten Verfahrens, ist jedoch nützlich für das Verständnis der Erfindung. Die Markierungen können aber auch, wie in Anspruch 10 beansprucht, an einem an das Gebiß des Patienten angepaßten Mundstück angebracht sein. Diese Markierungen werden mittels bildsignalerzeugender Sensoren 34, 36 nach Art von Videokameras bei der vorstehend beschriebenen ersten Ausrichtung beobachtet. Dadurch wird die Ausgangsposition bestimmt. Bei späteren Bestrahlungen werden die Markierungen von den raumfest gehaltenen Sensoren 34, 36 erfaßt. Aus den Bildinformationen der Sensoren wird eine Ist-Lage bestimmt. Durch einen Regelkreis mit Stellmotoren wird der Schädel des Patienten wieder in die Soll-Lage bewegt.

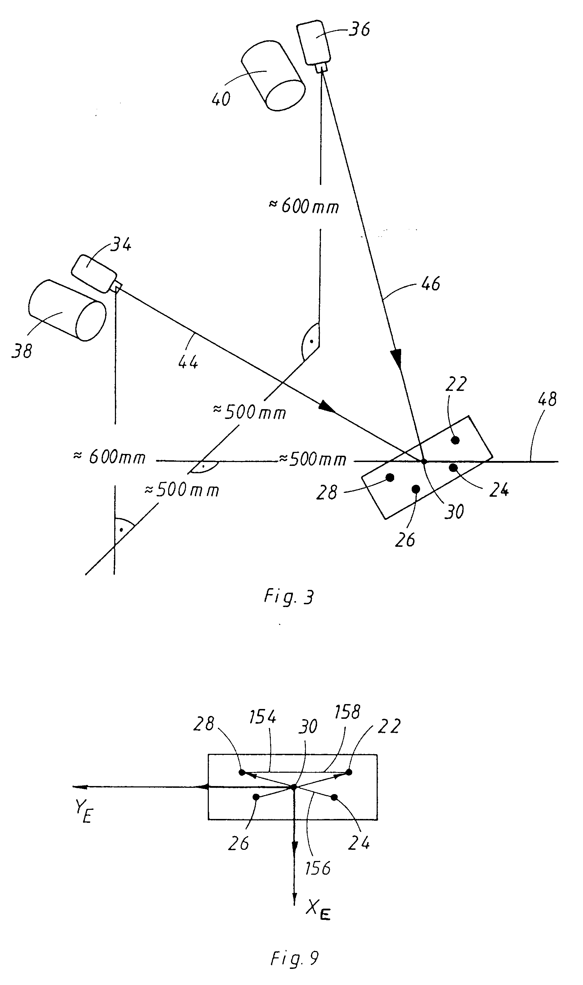

In Fig.3 ist ein Beispiel für solche Markierungen dargestellt. Es sind hier insgesammt vier Markierungen vorgesehen. Davon liegen vier Markierungen 22, 24, 26 und 28 etwa in den Ecken eines symmetrischen Trapezes. Mit 30 ist der Schwerpunkt der vier Markierungen 22, 24, 26, 28 bezeichnet. Die Markierungen sind von hellen Kugeln gebildet, die sich vor einem dunklen Hintergrund befinden.

Die Markierungen 22, 24, 26 und 28 werden von zwei bildsignalerzeugenden Sensoren 34 und 36 beobachtet. Durch Lampen 38 und 40 erfolgt eine möglichst gleichmäßige Beleuchtung der Markierungen von allen Seiten. Bei der dargestellten Ausführung sind die beiden Sensoren in einer vertikalen Ebene in einem horizontalen Abstand von etwa 500 mm und etwa 600 mm oberhalb der die Markierung 30 enthaltenden Horizontalebene angeordnet. Sie sind mit ihren optischen Achsen 44 bzw. 46 im wesentlichen auf den Schwerpunkt 30 ausgerichtet. Dieser Schwerpunkt 30 liegt in einem Abstand von etwa 500 mm von der die Sensoren 34 und 36 enthaltenen Vertikalebene in der zu dieser Vertikalebene senkrechten, vertikalen Symmetrieebene zwischen den Sensoren 34 und 36.

Die vorstehend angegebene Geometrie und die Maßangaben sind nicht kritisch. Sie geben einen Anhalt dafür, mit welcher Anordnung in der Praxis eine hinreichend genaue Ausrichtung des Patienten erfolgen kann.

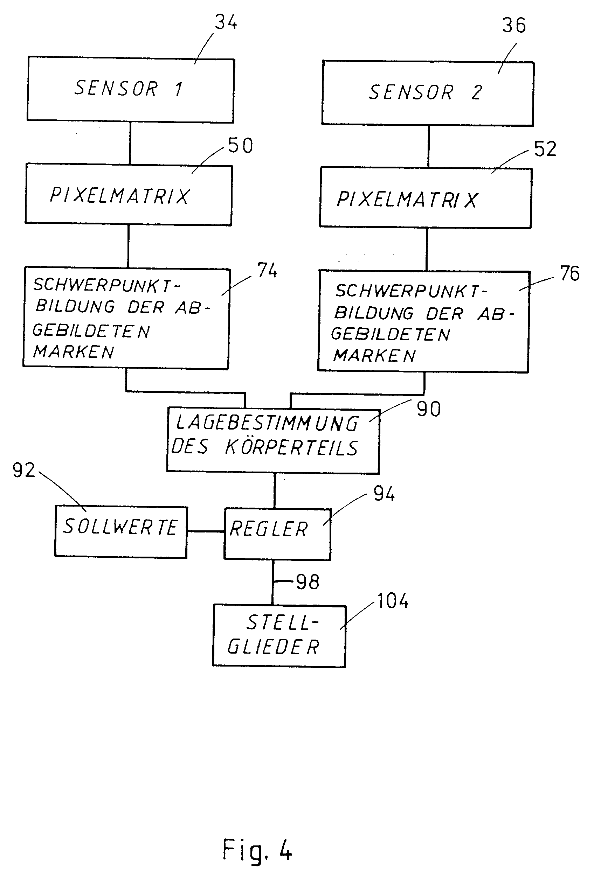

Die Verarbeitung der von den Sensoren 34 und 36 erhaltenen Bildinformationen ist in Fig.4 schematisch in einem Diagramm dargestellt.

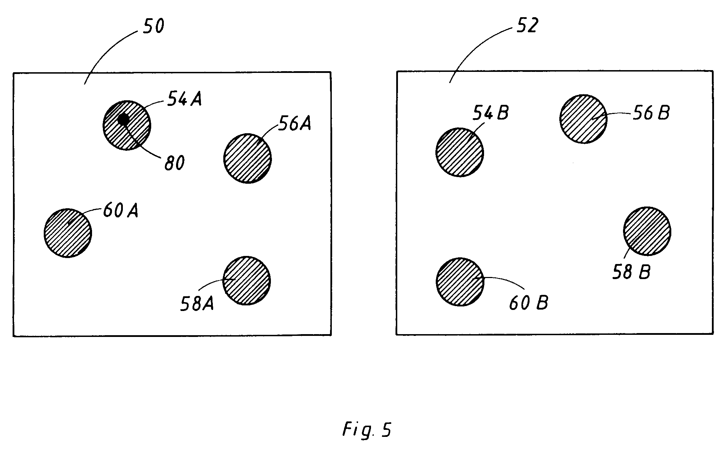

Die Sensoren 34 und 36 liefern eine Pixelmatrix 50 bzw. 52. Jedes Bildelement des von dem betreffenden Sensor 34 oder 36 erfaßten Bildes liefert einen Helligkeitswert. Fig.5 zeigt ein Beispiel für die von den Sensoren 34 und 36 gelieferten Pixelmatrizen 50 bzw. 52. Man erkennt in den Pixelmatrizen 50 und 52 kreisförmige Gebilde 54A, 56A, 58A, 60A bzw. 54B, 56B, 58B, 60B als Abbilder der Markierungen 22, 24, 26, 28. Die Markierungen sind Kugeln. Als Abbilder ergeben sich daher unabhängig von der Beobachtungsrichtung im wesentlichen Kreise.

Die Bildverarbeitung umfaßt nun folgende Schritte:

Es werden zunächst die Abbilder der Markierungen identifiziert. Diese Identifizierungs-Operation besteht darin, die verschiedenen Abbilder von Markierungen z.B. 54A bestimmten Markierungen z.B. 22 zuzuordnen.

Die Pixel stellen ein relativ grobes Raster dar. Die Abmessungen eines Pixels projiziert in die Ebene der Markierungen sind größer als die geforderte Positioniergenauigkeit. Natürlich sind auch die Abmessungen der Markierungen selbst wesentlich größer als die geforderte Positioniergenauigkeit. Der nächste Schritt besteht daher in einer Schwerpunktbildung. Es werden die Schwerpunkte der als Abbilder der Markierungen identifizierten Objekte gebildet. Die Koordinaten der Schwerpunkte können mit wesentlich größerer Genauigkeit als eine Pixellänge oder -höhe bestimmt werden.

Es ist also zur Erzielung einer Genauigkeit der Positionierung von Bruchteilen eines Millimeters weder erforderlich, entsprechend kleine Markierungen vorzusehen und abzutasten, noch ist es erforderlich, die Abtastung mit einer der geforderten Genauigkeit der Positionierung entsprechenden Auflösung vorzunehmen. Es genügen relativ große Markierungen, die allerdings definierte, regelmäßige Gestalt haben sollten, und Sensoren, welche das Bild mit einem relativ groben Raster erfassen.

Die Schwerpunktbildung ist in Fig.4 durch Blöcke 74 und 76 angedeutet.

Es sind jetzt in der Bildebene des Sensors 34 oder 36 Punkte 80 definiert, die den Schwerpunkten der Abbilder der Markierungen entsprechen. Aus den Punkten 80 und den Abbildungseigenschaften der Optik des Sensors 34 bzw. 36 (einschließlich der Abbildungsfehler) der Strahl 48 berechnet werden, der vom Sensor 34 oder 36 zu dem Schwerpunkt der Markierung (z.B. 22) verläuft.

Die Berechnung der Lage des Körperteils ist in Fig.4 durch den Block 90 dargestellt.

Um die erforderliche hohe Genauigkeit zu erreichen, ist es notwendig, zunächst die individuellen Abbildungseigenschaften jedes Sensors zu ermitteln. Dies geschieht durch einmalige Vermessung der Abbildung eines genau bekannten Testkörpers.

Weiterhin müssen der Ort und die Ausrichtung aller Sensoren im Koordinatensystem des Bestrahlungsgerätes in kleinen Zeitabständen (täglich) genau ermittelt werden. Dazu wird ein Referenzkörper mit seinem Mittelpunkt in das Isozentrum 20 gebracht. Die markierten Achsen des Referenzkörpers werden parallel zu den Achsen des auf das Bestrahlungsgerät 10 bezogenen, raumfesten Koordinatensystems ausgerichtet. Der Referenzkörper trägt fünf Markierungen. Diese Markierungen sind ebenfalls von Kugeln vor einem kontrastierenden Hintergrund gebildet. Die Lage der Markierungen zum Mittelpunkt des Referenzkörpers ist genau bekannt. Es wird dann die Lage des Sensors mit drei kartesischen Koordinaten und drei Lagewinkeln in bezug auf das Koordinatensystem des Referenzkörpers aus den Schwerpunkten der abgebildeten Markierungen berechnet.

Mathematisch ist es vorteilhaft, die Berechnung der Lage des Sensors mittels der inversen Funktion vorzunehmen: Es werden für eine vorgegebene, geschätzte Lage der Kamera die Schwerpunktkoordinaten der Abbilder berechnet. Diese Schwerpunktkoordinaten weichen in der Regel von den tatsächlich beobachteten Schwerpunktkoordinaten der Abbilder ab. Es wird dann die quadratische Abstandssumme zwischen berechneten und beobachteten Schwerpunktkoordinaten gebildet. Diese quadratische Abstandssumme wird durch Korrekturen der geschätzten Lage des Sensors minimiert. Für diese Korrekturen ergibt sich ein nichtlineares Gleichungssystem. Dieses Gleichungssystem wird durch Linearisierung und Iteration gelöst. Dadurch ergeben sich bestmögliche Werte für die Lage des Sensors im gerätefesten und referenzkörperfesten Koordinatensystem.

Bei der ersten Bestrahlung wird der zu bestrahlende Körperteil mittels stereotaktischer Verfahren, wie beschrieben, ausgerichtet und fixiert. Dann befindet sich der Behandlungspunkt (Tumor) genau im Isozentrum 20. In dieser Position werden die Positionen der am Körperteil angebrachten Markierungen mittels der Sensoren 34 und 36 im gerätefesten Koordinatensystem wie folgt ermittelt:

Aus der in der beschriebenen Weise bestimmten Lage der Sensoren und den an dem Körperteil des Patienten angebrachten Markierungen werden die Strahlen zu den Schwerpunkten der Markierungen berechnet. Das geschieht unter Benutzung der mit dem Testkörper gewonnenen Informationen über die durch den Sensor erfolgende Abbildung. Die berechneten Strahlen schneiden sich u.U. nicht exakt. Als Position einer Markierung wird dann der Punkt gewählt, der zu den beiden Strahlen, die sich für die beiden Sensoren 34 und 36 ergeben, einen minimalen Abstand hat.

Die so mittels der Sensoren 34 und 36 bestimmten Koordinaten der Markierungen 22, 24, 26, 28 definieren die Soll-Lage des Körperteils.

Bei jeder späteren Bestrahlung, bei welcher der zu bestrahlende Körperteil nicht exakt ausgerichtet und nicht starr fixiert ist, wird laufend die Lageabweichung von der Soll-Lage aus den momentanen Schwerpunktkoordinaten der abgebildeten Markierungen berechnet. Die Lageabweichung wird durch eine Verschiebung mit drei Translationsgrößen und eine Drehung mit drei Rotationswinkeln beschrieben.

Die Berechnung benutzt wieder wie bei der Vermessung der Sensoren die inverse Funktion: Es werden die Schwerpunkte der Abbilder der Markierungen 22, 24, 26, 28 in den Bildkoordinatensystemen der Sensoren 34 und 36 als Funktion der sechs Freiheitsgrade der Lageabweichung berechnet. Die Minimierung der quadratischen Abstandssumme zwischen berechneten und gemessenen Schwerpunktkoordinaten führt wieder zu einem nichtlinearen Gleichungssystem. Dieses Gleichungssystem wird durch Linearisierung und Iteration gelöst. Dadurch ergeben sich bestmögliche Werte für die Lageabweichung des Körperteils im gerätefesten Koordinatensystem und insbesondere für die Verschiebung des Behandlungspunktes aus der Soll-Lage.

Die Koordinaten der vier Markierungen liefern die Istlage eines "patientenfesten" Koordinatensystems. Diese Istlage wird mit der "Soll-Lage" verglichen, die bei der ersten genauen Positionierung bestimmt und in einem Speicher 92 gespeichert wurde. Ein Regler 94 ermittelt die Regelabweichung von Istlage und Soll-Lage und erzeugt Stellsignale an Ausgängen 98. Die Stellsignale sind auf Stellglieder aufgeschaltet, welche in Fig.4 durch Block 104 symbolisiert sind.

Im einzelnen wird folgendermassen vorgegangen:

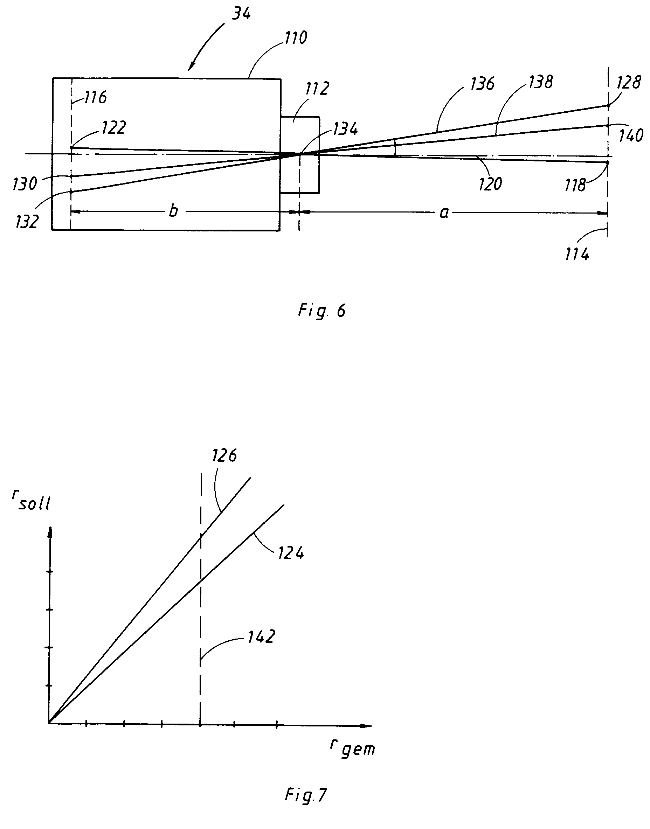

Zunächst werden die Abbildungseigenschaften jedes einzelnen Sensors bestimmt. Das wird anhand der Fig. 6 und 7 erläutert.

In Fig. 6 ist schematisch ein Sensor, z.B.34, dargestellt. Der bilderzeugende Sensor 34 hat die Form einer Kamera mit einem Kameragehäuse 110 und einem Objektiv 112. Das Objektiv 112 bildet ein ebenes Testobjekt 114 auf eine zweidimensionale Anordnung 116 von Sensorelementen ab. Das Testobjekt enthält ein Muster, z.B. ein Muster von konzentrischen Kreisen mit einem Mittelpunkt 118. Das Testobjekt 114 wird so zu dem Sensor 34 angeordnet, dass die Mittelachse 120 des Sensors 34 im wesentlichen durch den Mittelpunkt 118 geht. Es entsteht ein entsprechendes Muster auf der zweidimensionalen Anordnung 116 von Sensorelementen.

Durch Abbildungsfehler des Objektivs 112 ist die Abbildung etwas verzerrt. Das ist in Fig. 7 übertrieben dargestellt. Der Mittelpunkt 118 liegt u.U. durch Ausrichtfehler nicht genau in der Mitte der Anordnung 116 von Sensorelementen. Der Mittelpunkt 118 wird auf einen Fusspunkt 122 abgebildet. Auf diesen Fusspunkt 122 wird das von der Anordnung 116 von Sensorelementen erfasste Bild des Musters bezogen. Es wird angenommen, dass die Verzeichnung des Objektivs 112 rotationssymmetrisch ist, also nur von dem auf den Fusspunkt 122 bezogenen Radius abhängt. Bei idealer Abbildung wäre die Charakteristik von Fig. 7 eine unter 45° zu den $r_{gem}$- und $r_{soll}$-Koordinatenachsen verlaufende Gerade 124. Dabei ist "$r_{gem}$" der von den Sensorelementen "gemessene" Radius eines Bildpunktes des Musters des Testobjektes 114. Mit $r_{soll}$ ist der Radius bezeichnet, der sich bei idealer Abbildung ergeben sollte. Tatsächlich ist die Charakteristik 126 in Fig. 7 etwas nach oben gekrümmt.

Der gemessene Radius $r_{gem}$ ist etwas kleiner als er für eine ideale Abbildung sein sollte. Die Charakteristik 126 ist durch eine Gleichung von der Form

$$r_{soll} = r_{gem} + a_3 r_{gem}^3 + a_5 r_{gem}^5 + \cdots$$

darstellbar. Ein Punkt 128 des Musters wird durch das Objektiv 112 in Punkt 130 auf der Anordnung 116 von Sensorelementen abgebildet, statt in dem Punkt 132, der sich aus der durch den Hauptpunkt 134 des Objektivs 112 gehenden Geraden 136 ergibt. Der Punkt 130 würde mit dem Strahl 138 einem Punkt 140 zugeordnet.

Bei der Berechnung des Sehstrahls zu dem zugehörigen "Objektelement" werden diese Korrekturen berücksichtigt. Ein Helligkeitswert, der z.B. bei $r_{gem}= 4$ von dem entsprechenden Sensorelement gemessen wird, wird für die Berechnung des Sehstrahls einem Pixel mit $r_{soll}=5$ zugeordnet, wie in Fig. 7 längs der Linie 142 - stark übertrieben - dargestellt ist.

Durch einen Faktor $sk_y$ bzw. $sk_z$ kann weiterhin das Verhältnis von Strahlwinkel zu Bildpunktkoordinaten berücksichtigt werden. Es gibt für jede Koordinatenrichtung einen solchen Faktor. Es ergibt sich dann eine Darstellung, welche die Position der Punkte in der Gegenstandsebene getreu wiedergibt. In gleicher Weise wird mit dem Sensor 36 verfahren.

Die Koeffizienten $a_3$ und $a_5$ und der vorerwähnte Faktor stellen "innere Kamera-Parameter" für die beiden Sensoren 34 und 36 dar. Sie werden einmal bestimmt und in den Rechner für die Signalverarbeitung eingegeben. Es kann damit jedem Sensorelement ein entsprechender Sehstrahl zugeordnet werden.

Der nächste Schritt ist die Initialisierung der Lage, also der Position und Orientierung der beiden Sensoren 34 und 36. Position und Orientierung der Sensoren 34 und 36 werden in einem Koordinatensystem gemessen, das fest zu der Bestrahlungsvorrichtung 10 ausgerichtet ist. Zweckmässigerweise wird der Ursprung des Koordinatensystems in das Isozentrum 20 gelegt. Eine Koordinatenachse $x_l$ fluchtet mit der Schwenkachse 18 der Bestrahlungsvorrichtung, eine Koordinatenachse $z_l$ ist vertikal, und die dritte Koordinatenachse $y_l$ verläuft senkrecht zu den Koordinatenachsen $x_l$ und $z_l$. Das ist in Fig. 1 dargestellt.

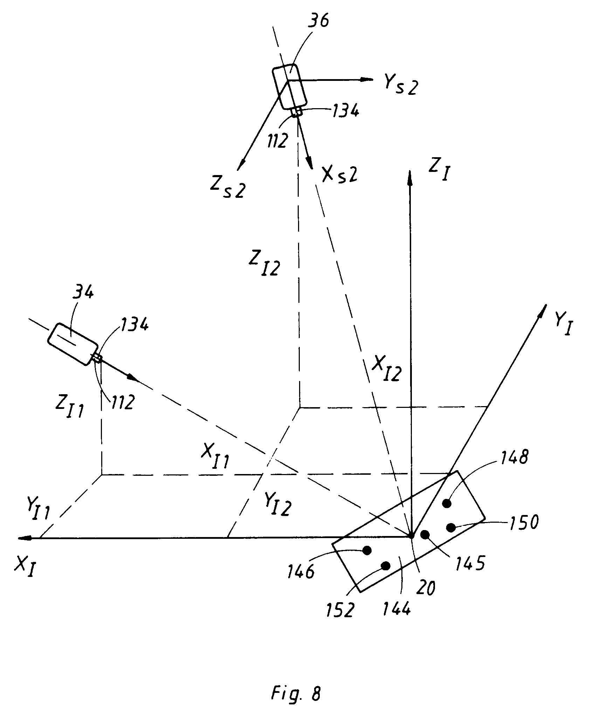

In Fig. 8 ist das Koordinatensystem $x_l$, $y_l$ und $z_l$ dargestellt. Im Bereich des Isozentrums 20 ist ein Referenzkörper 144 derart angeordnet, dass er von den Sensoren 34 und 36 voll erfasst wird. Der Referenzkörper 144 weist fünf Markierungen 145, 146, 148, 150 und 152 auf. Die Markierungen 145, 146, 148, 150 und 152 sind ähnlich wie die Markierungen 22 bis 28 von Kugeln gebildet, die von den Sensoren 34 und 36 unabhängig von der Beobachtungsrichtung als kreisförmige Gebilde gesehen werden. Die Lage des Referenzkörpers 144 und die Positionen der Markierungen 145 bis 152 in dem Koordinatensystem $x_l$, $y_l$, $z_l$ sind genau bekannt. Diese Positionen der Markierungen werden in den Rechner eingegeben.

Es erfolgt dann eine Umschaltung auf "Livebilder". Die von den Sensorelementen erfassten Bilder erscheinen unmittel-bar auf einem Monitor. Anhand dieser Bilder können die Sensoren 34 und 36 so ausgerichtet werden, dass jeder Sensor 34 und 36 alle fünf Markierungen 145 bis 152 erfasst. Anhand der Bilder auf dem Monitor und eines mittels einer Maus auf die Bilder der Markierungen 145 bis 152 geführten Cursors werden die Markierungen identifiziert und numeriert (Voreinweisung). Es können auch grob die Pixelwerte für die Schwerpunkte der Bilder der Markierungen bestimmt werden.

Jeder der Sensoren 34 und 36 liefert jetzt ein Bild, in dem die fünf Markierungen 145 bis 152 als kreisförmige Gebilde erscheinen.

Dazu werden ausgehend von dem Pixel, das bei der Voreinweisung der Markierungen 145 bis 152 für eine solche Markierung bestimmt wurde und im Inneren des Bildes der Markierung liegt, in beiden Koordinatenrichtungen des sensorfesten Koordinatensystems Zeile für Zeile und Spalte für Spalte anhand der Grauwerte des Bildes die Orte der Übergänge von den z.B. weissen Markierungen zu dem dunklen Hintergrund gesucht. Das geschieht mittels einer Grauwertschwelle und einer subpixelgenauen Grauwert-Interpolation. Aus den so gefundenen Rändern wird dann für jede Koordinatenrichtung getrennt der Schwerpunkt oder Mittelpunkt des Bildes der Markierung z.B. 145 gebildet.

Das ist bekannte Bildverarbeitungstechnik und daher hier nicht im einzelnen beschrieben.

Es ergeben sich somit für jeden Sensor 34 und 36 fünf Mittelpunkte der Bilder der Markierungen 145 bis 152 mit je zwei Koordinaten in dem sensorfesten Koordinatensystem. In diesem Koordinatensystem liegen zwei Koordinatenachsen $y_s$ und $z_s$ in der Ebene der zweidimensionalen Anordnung 116 (Fig. 6) von Sensorelementen und jeweils parallel zu deren Zeilen bzw. Spalten. Die dritte Koordinatenachse $x_s$ verläuft in Richtung der optischen Achse 120 des Sensors 34 bzw. 36. Das ist in Fig. 8 angedeutet.

Die so erhaltenen Mittelpunkte der Bilder der Markierungen mit den Koordinaten $y_{si}\, z_{si}$ (i=1...5) werden mittels der inneren Kamera-Parameter korrigiert.

Die Lage der Sensoren 34 und 36 ist durch je sechs Lageparameter bestimmt, nämlich durch die drei kartesischen Koordinaten $x_{l1}$, $y_{l1}$, $z_{l1}$ bzw. $x_{l2}$, $y_{l2}$, und $z_{l2}$ und drei Eulerwinkel $\psi_1$, $\vartheta_1$, $\varphi_1$ bzw. $\psi_2$, $\vartheta_2$, $\varphi_2$. Die kartesischen Koordinaten der beiden Sensoren 34 und 36 sind in Fig. 8 eingezeichnet. Als kartesischen Koordinaten eines Sensors 34 oder 36 werden dabei die Koordinaten des Hauptpunktes 134 des Objektivs 112 jedes Sensors 34 oder 36 verwendet. Die kartesischen Koordinaten der Sensoren 34 und 36 können zu Ortsvektoren

$$\underline{S}_1 = (x_{l1}, y_{l1}, z_{l1}) \ bzw. \ \underline{S}_2 = (x_{l2}, y_{l2}, z_{l2})$$

zusammengefasst werden. Die jeweils sechs Lageparameter liefern Lagevektoren

$$\underline{v}_1 = (x_{l1}, y_{l1}, z_{l1}, \psi_1, \vartheta_1, \varphi_1)$$

bzw.

$$\underline{v}_2 = (x_{l2}, y_{l2}, z_{l2}, \psi_2, \vartheta_2, \varphi_2)$$

Dabei ist jeweils (mit m = 1,2)

$\varphi_m$          die Rotation um die Achse $x_l$ des ortsfesten Koordinatensystems
$\vartheta_m$          die Rotation um die Achse $y_l$ des ortsfesten Koordinatensystems
$\psi_m$          die Rotation um die Achse $z_l$ des ortsfesten Koordinatensystems

für die Sensoren 34 (m=1) und 36 (m=2).

Die drei Rotationen können durch drei Transformationsmatrizen oder Richtungskosinus-Matrizen $R_z(\psi)$, $R_y(\vartheta)$ und $R_x(\varphi)$ beschrieben werden.

Es ist

$$R_Z(\psi_m) = \begin{pmatrix} \cos\psi_m & -\sin\psi_m & 0 \\ \sin\psi_m & \cos\psi_m & 0 \\ 0 & 0 & 1 \end{pmatrix}$$

$$R_Y(\vartheta_m) = \begin{pmatrix} \cos\vartheta_m & 0 & \sin\vartheta_m \\ 0 & 1 & 0 \\ -\sin\vartheta_m & 0 & \cos\vartheta_m \end{pmatrix}$$

$$R_X(\varphi_m) = \begin{pmatrix} 1 & 0 & 0 \\ 0 & \cos\varphi_m & -\sin\varphi_m \\ 0 & \sin\varphi_m & \cos\varphi_m \end{pmatrix}$$

Die Gesamtrotation wird dargestellt durch eine Matrix

$$R_m = R_z(\psi_m) R_y(\vartheta_m R_x(\varphi_m)$$

Das sind die Transformationen, welche Koordinaten aus dem jeweiligen Sensorkoordinatensystem $x_{s1}, y_{s1}, z_{s1}$ für den Sensor 34 bzw. $x_{s2}, y_{s2}, z_{s2}$ für den Sensor 36 in das ortsfeste Koordinatensystem transformieren. Das Sensorkoordinatensystem z.B. des Sensors 36 kann durch drei Einheitsvektoren $\underline{E}_{x2}, \underline{E}_{y2}, \underline{E}_{z2}$ in Richtung der drei Koordinatenachsen $x_{s2}, y_{s2}, x_{s2}$ definiert werden. In dem Sensorkoordinatensystem haben diese drei Einheitsvektoren die Form

$$\underline{E}_{X2}^S = \begin{pmatrix} 1 \\ 0 \\ 0 \end{pmatrix}, \quad \underline{E}_{Y2}^S = \begin{pmatrix} 0 \\ 1 \\ 0 \end{pmatrix}, \quad \underline{E}_{Z2}^S = \begin{pmatrix} 0 \\ 0 \\ 1 \end{pmatrix}$$

Entsprechendes gilt für das Koordinatensystem des Sensors 34. In dem ortsfesten Koordinatensystem $x_l$, $y_l$, $z_l$ wird dann

$$\underline{E}_{X2}^l = R\begin{pmatrix} 1 \\ 0 \\ 0 \end{pmatrix}, \quad E_{Y2}^l = R\begin{pmatrix} 0 \\ 1 \\ 0 \end{pmatrix}, \quad E_{Z2}^l = R\begin{pmatrix} 0 \\ 0 \\ 1 \end{pmatrix}$$

Mit $\underline{M}_i$ (i = 1...5) sei in dem ortsfesten Koordinatensystem der Ortsvektor des Mittelpunktes der i-ten Markierung 145 bis 152 bezeichnet. Dieser Ortsvektor ist genau bekannt. $\underline{S}_m$ (m = 1 ; 2) ist der geschätzte, also nicht genau bekannte Ortsvektor des Sensors 34 oder 36. Auch die Matrix R mit den Winkel funktionen der Euler-Winkel ist geschätzt, also nicht genau bekannt, und das gilt dann natürlich auch für die Einheitsvektoren $\underline{E}_{xm}^l$, $\underline{E}_{ym}^l$, $E_{zm}^l$ bezo-

gen auf das ortsfeste Koordinatensystem $x_I$, $y_I$, $z_I$. Es wird berechnet, welche Koordinaten die Mittelpunkte der Bilder der Markierungen 145 bis 152 unter der Annahme einer solchen Lage der Sensoren 34 und 36 in den sensorfesten Koordinatensystemen haben würden. Diese Lage ist gegeben durch

$$\hat{Y}_i = sk_y \; \frac{\underline{E}^I_{Ym} \cdot (\underline{M}_i - \underline{S}_m)}{\underline{E}^I_{Xm} \cdot (\underline{M}_i - \underline{S}_m)}$$

$$\hat{Z}_i = sk_z \; \frac{\underline{E}^I_{Zm} \cdot (\underline{M}_i - \underline{S}_m)}{\underline{E}^I_{Xm} \cdot (\underline{M}_i - \underline{S}_m)}$$

Die Vektordifferenz $\underline{M}_i - \underline{S}_m$ ist der Vektor von dem Hauptpunkt 134 des Objektivs 112 zu einer Markierung "i" des Referenzkörpers 144. Die skalare Multiplikation dieser Vektordifferenz mit dem Einheitsvektor in $y_s$-Richtung liefert die Komponente dieser Vektordifferenz in $y_s$-Richtung. Die skalare Multiplikation der Vektordifferenz $\underline{M}_i - \underline{S}_m$ mit dem Einheitsvektor $\underline{E}^S_{xm}$ liefert die Komponente der Vektordifferenz in Richtung der optischen Achse 120 des Sensors 34 oder 36. Das Verhältnis der skalaren Produkte ist näherungsweise der Winkel, den die Vektordifferenz mit der optischen Achse 120 des Sensors 34 oder 36 bildet. Multipliziert mit dem Faktor $sk_y$ ergibt sich die $y_s$-Koordinate $y_i$ des sich aus der geschätzten Lage des Sensors 34 oder 36 berechneten Bildpunktes.

Entsprechend erfolgt die Berechnung der Koordinate $z_i$.

Beobachtet werden aber Koordinaten $y_{i\,korr}$ und $z_{i\,korr}$. Das sind die Koordinaten der den Mittelpunkten der Bilder der Markierungen 145 bis 152 entsprechenden Sensorelemente, korrigiert - wie oben erläutert - hinsichtlich der inneren Kamera-Parameter.

Es ergeben sich für jeden Sensor 34 und 36 Fehler-Vektoren mit den Komponeten:

$$f_{iy} = sk_y \; \cdot \; \frac{\underline{E}^I_{Ym} (\underline{M}_i - \underline{S}_m)}{\underline{E}^I_{Xm} (\underline{M}_i - \underline{S}_m)} - y_{i\,korr}$$

$$f_{iz} = sk_z \; \cdot \; \frac{\underline{E}^I_{Zm} (\underline{M}_i - \underline{S}_m)}{\underline{E}^I_{Xm} (\underline{M}_i - \underline{S}_m)} - z_{i\,korr}$$

Die angenommene Lage (Position $\underline{S}_m$ und Orientierung $\underline{R}_m$) des Sensors 34 bzw. 36 entspricht im allgemeinen zunächst nicht der wahren Lage. Es treten die genannten Fehlervektoren auf. Im vorliegenden Fall hat der gesuchte Vektor $\underline{v}$ sechs Komponenten. Die Fehlervektoren liefern insgesamt zehn Fehler. Es gilt nun, die Komponenten des Vektors $\underline{v}$, also z.B. für Sensor 36 die Koordinaten $x_{I2}$, $y_{I2}$, $z_{I2}$ und die Euler-Winkel $\psi$, $\vartheta$, $\varphi$ gezielt so zu variieren, dass die Fehler verschwinden. Das kann mittels der bekannten Newtonschen Iteration geschehen.

Es wird aus den Fehlervektoren eine Fehlerfunktion

$$F = f^2_{1y} + f^2_{2y} + f^2_{3y} + f^2_{4y} + f^2_{5y} + f^2_{1z} + f^2_{2z} + f^2_{3z} + f^2_{4z} + f^2_{5z}$$

gebildet. Diese Fehlerfunktion soll minimiert werden. Das entspricht einem "Least-Square-Fit".

Für das gesuchte Minimum gilt:

grad $F = \underline{0}$,

wobei $\underline{0}$ der Nullvektor ist. Die Komponenten der Fehlervektoren $f_{iy}$ und $f_{iz}$ hängen jede von allen Komponenten des Vektors $\underline{S}$ nach bekannten Funktionen ab. Es können daher die partiellen Ableitungen

$$D_{kl} = \frac{\partial f_i}{\partial v_l}$$

gebildet werden, wobei $v_l$ die 1-te Komponente des Vektors $\underline{v}$ ist. Es ist also etwa für Sensor 36 $v_2 = y_{I2}$. Es ergibt sich eine "Jacobi-Matrix" D, die im vorliegenden Fall eine 10 x 6Matrix ist. Mit dieser Jacobi-Matrix ergibt sich ein Iterationsschritt zur Lösung der Gleichung grad $F = \underline{0}$ in der Form:

$$\underline{v}_{(n+1)} = \underline{v}_{(n)} - (D^T D)^{-1} D^T \underline{f}$$

Darin sind $\underline{v}_{(n+1)}$ und $\underline{v}_{(n)}$ die sich aus dem (n+1)ten oder n-ten Iterationsschritt ergebenden Näherungen für den Vektor $\underline{v}$. $D^T$ ist die transponierte Jacobi-Matrix D, also eine Matrix, bei welcher gegenüber der Matrix D Zeilen und Spalten vertauscht sind. Das Zeichen $^{-1}$ bedeutet die inverse Matrix. Und $\underline{f}_{(n)}$ ist der Fehlervektor nach dem n-ten Iterationsschritt.

Die Iteration wird fortgesetzt, bis der Fehlervektor $\underline{f}_{(n)}$ einen vorgegebenen Schwellwert unterschreitet. Dann ist die Lage des betreffenden Sensors 34 oder 36 hinreichend genau bekannt. Das beschriebene Verfahren wird für die beiden Sensoren 34 und 36 getrennt durchgeführt.

Die erhaltenen Daten über die Lage der Sensoren 34 und 36 werden gespeichert. Diese Daten werden anschliessend für die Positionierung von Patienten benutzt. Die Bestimmung der Lage der Sensoren wird in regelmässigen Zeitabständen, z.B. täglich, wiederholt.

Bei der Positionierung eines Patienten sind z.B. an dem Kopf des Patienten vier kugelförmige Markierungen 22, 24, 26, 28 in definierter Lage angebracht. Bei der ersten Vermessung ist der Kopf des Patienten, wie beschrieben, mittels stereotaktischer Verfahren in eine solche Lage gebracht, dass sich ein zu bestrahlender Tumor genau in dem Isozentrum 20 befindet. In dieser Lage werden die vier Markierungen 22, 24, 26 und 28 durch die beiden Sensoren 34 und 36 vermessen.

Zu diesem Zweck werden ähnlich wie bei den Markierungen 145, 146, 148, 150 und 152 des Referenzkörpers 144 zunächst die Schwerpunkte der Bilder dieser Markierungen 22, 24, 26 und 28 bestimmt. Diese Schwerpunkte werden nach Massgabe der inneren Kamera-Parameter (Fig. 7) korrigiert, wie dies oben im Zusammenhang mit den Markierungen 145, 146, 148, 150 und 152 des Referenzkörpers schon beschrieben wurde. Zu jedem der so bestimmten, korrigierten Schwerpunkte gehört ein "Sehstrahl", der von diesem Schwerpunkt durch den Hauptpunkt 134 des Objektivs 112 verläuft. Dieser Sehstrahl sollte durch den Mittelpunkt der Markierung hindurchgehen.

Zunächst wird für jede der Markierungen 22, 24, 26 und 28 unabhängig die absolute Position im Raum in dem ortsfesten Koordinatensystem $x_l, y_l, z_l$ bestimmt. Zu diesem Zweck wird zunächst ein Startwert ermittelt als "Schnittpunkt" der von den beiden Sensoren 34 und 36 auf ein und dieselbe Markierung gerichteten Sehstrahlen. Aufgrund von Messungenauigkeiten haben die Sehstrahlen im Raum im allgemeinen keinen exakten Schnittpunkt. Daher wird als "Schnittpunkt" jeweils der Mittelpunkt der Abstandsstrecke zwischen den Sehstrahlen gewählt.

Es wird nun wieder ausgehend von den Startwerten die genaue Position der Markierungen 22, 24, 26 und 28 im ortsfesten Koordinatensystem $x_l, y_l, z_l$ für jede Markierung einzeln mittels einer Newton-Iteration bestimmt. Der als Schnittpunkt der Sehstrahlen geschätzte Positionsvektor $\underline{P}_j$ der j-ten Markierung 22, 24, 26 oder 28 (j = 1...4) im Koordinatensystem $x_l, y_l, z_l$ ist

$$\underline{P}_j (x_j, y_j, z_j).$$

Es ergeben sich dann im sensorfesten Koordinatensystem für den ersten Sensor 34 (m=1) die Fehler, also Abweichungen zwischen berechneter und beobachteter Lage des Bildes der Markierung

$$f_{y1}^i = sk_{y1} \cdot \frac{\underline{E}_{Y1}^I \cdot (P_i - S_1)}{\underline{E}_{X1}^I \cdot (P_i - S_1)} - Y_{1\,korr}^i$$

$$f_{z1}^i = sk_{z1} \cdot \frac{\underline{E}_{Z1}^I \cdot (P_i - S_1)}{\underline{E}_{X1}^I \cdot (P_i - S_1)} - Z_{1\,korr}^i$$

Ebenso ergeben sich im sensorfesten Koordinatensystem für den zweiten Sensor (m=2) die Fehler

$$f_{y2}^i = sk_{y2} \cdot \frac{\underline{E}_{Y2}^I (P_i - S_2)}{\underline{E}_{X2}^I (P_i - S_2)} - y_{2\,korr}^i$$

$$f_{Z2}^i = sk_{z2} \cdot \frac{\underline{E}_{Z2}^I (P_i - S_2)}{\underline{E}_{X2}^I (P_i - S_2)} - z_{2\,korr}^i$$

Dabei sind - ähnlich wie bei der Messung der Lage der Sensoren 34 und 36 mittels des Testkörpers 144 - $\underline{S}_1$ und $\underline{S}_2$ Lagevektoren der Sensoren 34 bzw. 36 und $y_{mkorr}$ und $Z_{mkorr}$ die beobachteten und hinsichtlich der inneren Kamera-Parameter korrigierten Koordinaten der Bilder der Markierungen 145 bis 152 im sensorfesten Koordinatensystem. Die Fehler sind wieder bekannte Funktionen der geschätzten Positionskoordinaten $x_j$, $y_j$ und $z_j$ ($P_1,P_2, P_3$). Die Fehler können für jede der Markierungen zu einem Fehlervektor

$$f = ( f_{y1}^{i}, f_{z1}^{i}, f_{y2}^{i}, f_{z2}^{i} ) = ( f_1, f_2, f_3, f_4 )$$

zusammengefasst werden. Es kann dann eine Jacobi-Matrix D* mit den Elemten

$$D_{ik}^{*} = \frac{\partial f_i}{\partial P_k}$$

gebildet werden. Die Jacobi-Matrix ist hier eine 4x3-Matrix.

Mit einer Newtonschen Iteration

$$\underline{P}_{j(n+1)} = \underline{P}_{j(n)} - (D^{*T} \cdot D^*)^{-1} \cdot D^{*T} \cdot \underline{f}$$

ergeben sich die Ortsvektoren der Markierungen 22, 24, 26 und 28 in dem ortsfesten Koordinatensystem $x_l$, $y_l$, $x_l$.

Aus den so erhaltenen Positionen der vier Markierungen 22, 24, 26, 28 wird ein "Eigenkoordinatensystem" $x_E$, $y_E$, $z_E$ definiert. Der Ursprung dieses Eigenkoordinatensystems liegt im Schwerpunkt 30 der vier Markierungen 22, 24, 26 und 28. Der in $z_E$-Richtung zeigende Einheitsvektor ist das normierte Vektorprodukt der beiden Vektoren 154 und 156, welche die Schwerpunkte der Markierungen 24 und 26 bzw. 28 und 22 miteinander verbinden (Fig. 9). Dieser Einheitsvektor und die $z_E$-Achse stehen somit senkrecht auf der Papierebene von Fig. 9 und sind in diese Papierebene hinein gerichtet. Der in Richtung der $y_E$-Achse zeigende Einheitsvektor läuft parallel zu der Verbindungslinie 158 zwischen den Schwerpunkten der Markierungen 22 und 28. Die $y_E$-Achse zeigt dabei nach links in Fig. 9. Der in Richtung der $x_E$-Achse zeigende Einheitsvektor bildet mit den Einheitsvektoren der $y_E$- und $z_E$-Achsen ein Rechtssystem und zeigt nach unten in Fig. 9.

Die so erhaltenen Daten $\underline{P}_j$ werden gespeichert. Wenn sich die Markierungen 22, 24, 26 und 28 in dieser Position befinden, dann befindet sich der Tumor genau im Isozentrum 20.

Bei einer nachfolgenden Bestrahlung liegen die bei stereotaktischer Ausrichtung des Kopfes einmal gespeicherten Koordinaten der Markierungen 22, 24, 26 und 28 vor. Es muss jetzt die Lage des Kopfes relativ zu diesen festen Koordinaten laufend bestimmt werden. Dies geschieht wieder mittels einer Newtonschen Iteration.

In diesem Fall ist ein Vektor

$$k = (\Delta X_k, \Delta Y_k, \Delta Z_k, \Delta \psi_k, \Delta \vartheta_k, \Delta \psi_k)$$

gesucht. Komponenten dieses Vektors sind die Abweichungen der Lage des kopffesten, durch die Markierungen 22, 24, 26 und 28 bestimmten Eigenkoordinatensystems von der Lage, die in der oben beschriebenen Weise bestimmt und gespeichert wurde und bei welcher der Kopf des Patienten in der durch stereotaktische Methoden festgelegten Lage mit dem Tumor im Isozentrum 20 gehalten wurde.

Man kann die Position des Kopfes bzw. des kopffesten Eigenkoordinatensystems durch einen Translationsvektor $\underline{t} = (\Delta x_k, \Delta y_k, \Delta z_k)$ angeben. Die Orientierung des Kopfes bzw. des kopffesten Eigenkoordinatensystems $x_E$, $y_E$, $z_E$ relativ zu dem ortsfesten Koordinatensystem $x_l$, $y_l$, $z_l$ kann durch eine Richtungskosinusmatrix $R_k$ als Funktion der Euler-Winkel $\Delta\psi_k$, $\Delta\vartheta_k$, $\Delta\varphi_k$ angegeben werden. Es ist wieder

$$R_k = R_{kz} (\Delta\psi_k) \cdot R_{ky} (\Delta\vartheta_k) \cdot R_{kx} (\Delta\varphi_k)$$

analog zu der oben angegebenen Richtungskosinusmatrix für die Festlegung der Orientierung des Sensors 36.

Für die Fehler in den sensorfesten Koordinatensystemen ergibt sich für die vier Markierungen 22, 24, 26 und 28 (j = 1...4) für den Sensor 34 (m = 1)

$$f_{y1}^{i} = sk_{y1} \cdot \frac{\underline{E}_{Y1}^{I} \cdot (R_k \cdot \underline{P}_i + \underline{t}) - \underline{S}_1)}{\underline{E}_{X1}^{I} \cdot (R_k \cdot \underline{P}_i + \underline{t}) - \underline{S}_1)} - Y_{1\,korr}^{i}$$

$$f_{z1}^{i} = sk_{z1} \cdot \frac{\underline{E}_{Z1}^{I} \cdot (R_k \cdot \underline{P}_i + \underline{t}) - \underline{S}_1)}{\underline{E}_{X1}^{I} (R_k \cdot \underline{P}_i + \underline{t}) - \underline{S}_1)} - Z_{1\,korr}^{i}$$

Für den Sensor 36 (m = 2) ergibt sich dementsprechend:

$$f_{y2}^{i} = sk_{y2} \cdot \frac{\underline{E}_{Y2}^{I} (R_k \cdot \underline{P}_i + \underline{t}) - \underline{S}_2)}{\underline{E}_{X2}^{I} (R_k \underline{P}_i + \underline{t}) - \underline{S}_2)} - Y_{2\,korr}^{i}$$

$$f_{z2}^{i} = sk_{z2} \cdot \frac{\underline{E}_{Z2}^{I} (R_k \underline{P}_i + \underline{t}) - \underline{S}_2)}{\underline{E}_{X2}^{I} (R_k \underline{P}_i + \underline{t}) - \underline{S}_2)} - Z_{2\,korr}^{i}$$

Darin stellt $R_k\underline{P}_j$ den um die Winkel $\Delta\psi_k$, $\Delta\vartheta_k$, $\Delta\varphi_k$ gedrehten Ortsvektor der j-ten Markierung (22, 24, 26 oder 28) dar. Dem so verdrehten Ortsvektor ist noch die Translation $\underline{t}$ überlagert. Es ergibt sich daraus mit einer geschätzten Richtungskosinusmatrix und einem geschätzten Translationsvektor $\underline{t}$ ein Schätzwert für die Lage jeder der Markierungen 22, 24, 26 oder 28. Die Differenz mit dem Vektor $\underline{S}_1$ oder $\underline{S}_2$ ergibt den Vektor von dem Hauptpunkt 134 des Objektivs 112 zu der Markierung "j", also 22, 24, 26 oder 28. Dieser Differenzvektor wird mit den Einheitsvektoren $E_{ym}$ bzw. $E_{zm}$ und dem Einheitsvektor $\underline{E}_{xm}$ skalar multipliziert. Die Verhältnisse liefern Winkel, die mit einem Skalenfaktor $sk_{ym}$ bzw. $sk_{zm}$ die berechneten Koordinaten der Bilder der Markierungen 22, 24, 26 oder 28 auf der Anordnung 116 von Sensorelementen in dem sensorfesten Koordinatensystem liefert. Die Differenz zu den tatsächlich beobachteten Bildern (mit Korrektur hinsichtlich der inneren Kamera-Parameter) liefern die Fehler.

Der Fehlervektor $\underline{f}$ hat bei zwei Sensoren 34 und 36 (m=1;2) und vier Markierungen 22, 24, 26, 28 (j = 1...4) mit je zwei Koordinaten sechzehn Komponenten. Die sechzehn Komponenten des Fehlervektors $\underline{f}$ sind bekannte Funktionen der sechs Komponeten des Vektors $\underline{k}$. Man kann wieder eine Jacobi-Matrix D' aus den partiellen Ableitungen der Komponenten des Fehlervektors $\underline{f}$ nach jeweils einer Komponente des Vektors $\underline{k}$ bilden. Eine solche Jacobi-Matrix ist eine 16 x 6 - Matrix.

Es werden nun zunächst zur Durchführung der Newtonschen Iteration als Schnitt zweier Sehstrahlen Schätzwerte für die Positionen der Markierungen 22, 24, 26 und 28 ermittelt. Aus diesen Schätzwerten ergeben sich Ursprung und Koordinatenrichtungen des Eigenkoordinatensystems $x_E$, $y_E$, $z_E$ der Markierungen 22, 24, 26 und 28. Aus diesem so ermittelten, aktuellen Eigenkoordinatensystem und dem in der Ursprungslage bestimmten, gespeicherten Eigenkoordinatensystem werden Rotation und Translation der Lageänderung ermittelt, also die Abbildung, welche das Eigenkoordinatensystem der Ausgangslage auf das aktuelle Eigenkoordinatensystem abbildet. Das liefert als Startwerte Komponenten des Vektors $\underline{k}$.

Es werden dann ausgehend von den Startwerten des Vektors $\underline{k}$ mittels der oben beschriebenen Newtonschen Iteration die tatsächlichen Komponenten des Vektors $\underline{k}$ berechnet.

Die vorstehend beschriebenen Operationen werden zyklisch wiederholt, so dass ständig der aktuelle Vektor $\underline{k}$ zur Verfügung steht. Dabei bildet dann das Resultat jedes Zyklus die Startwerte für den nächstfolgenden Zyklus.

Wie in Fig.1 schematisch dargestellt ist, liegt der Patient auf einem Schlitten 15, der in waagerechter Längsrichtung auf einem Bestrahlungstisch 16 verschiebbar geführt ist. Der Kopf des Patienten liegt auf einer Kopfauflage 108. Die Kopfauflage 108 ist relativ zu dem Schlitten in Vertikal- und in Querrichtung beweglich. In Querrichtung ist die Kopfauflage 108 durch ein Stellglied 160 verstellbar. In Vertikalrichtung ist die Kopfauflage 108 durch ein Stellglied 162 verstellbar. Eine Verstellung der Kopfauflage 108 in Längsrichtung relativ zu dem Schlitten 15 oder Bestrahlungstisch 16 ist nicht vorgesehen, da dann der Hals des Patienten bei einer Verstellung gedehnt oder gestaucht werden müßte. Stattdessen erfolgt in Längsrichtung eine Verstellung des Schlittens 15 durch ein Stellglied 164. Der Kopf des Patienten kann mit üblichen Mitteln auf der Kopfauflage 108 fixiert sein. Eventuelle Bewegungen werden aber durch die Regelkreise herausgeregelt. Der "Tumorpunkt" im Kopf des Patientenbleibt mit hoher Genauigkeit im Isozentrum 20.

Die beschriebene Anordnung kann in verschiedener Weise abgewandelt werden. Statt zweier Sensoren 34 und 36 können drei Sensoren vorgesehen werden. Bei Überbestimmung können dann die Koordinaten aus den berechneten Strahlen 44,46.. nach der Methode der kleinsten Fehlerquadrate berechnet werden.

EP 0 560 331 B1

**Patentansprüche**

1. Vorrichtung zur Positionierung eines Körperteils zur Behandlung mit einem medizinischen Gerät (10), enthaltend

   (a) Mittel (22,24,26,28,38,40) zum Erzeugen von optischen Informationen der Position des Körperteils relativ zu dem medizinischen Gerät (10) ,

   (b) mindestens zwei bildsignalerzeugende Sensoren (34,36), welche zur Beobachtung der optischen Informationen ausgerichtet sind,

   (c) bildverarbeitende Mittel (74,76) zur Verarbeitung der aus den optischen Informationen erzeugten Bildsignale (54A,56A,58A,60A,54B,56B,58B,60B) der Sensoren (34,36),

   (d) Signalverarbeitungs- und Reglermittel (90,92,94)

   -   zum Ermitteln der Istposition des Körperteils aus den beobachteten optischen Informationen,

   -   zum Vergleich der Istposition mit einer Sollposition und

   -   zur Erzeugung von Stellsignalen, die von der Regelabweichung zwischen Istposition und Sollposition abhängen, und

   (e) Mittel (104;160,162,164), die von den Stellsignalen beaufschlagt sind, zur Positionierung des Körperteils, so daß ein genau definierter Behandlungspunkt in einer Sollposition gehalten wird, und zur Verhinderung einer Behandlung bei Fehlausrichtung zwischen Gerät (10) und Körperteil,
   **dadurch gekennzeichnet, daß**

   (f) die Mittel (22,24,26,28,38,40) zum Erzeugen von optischen Informationen der Position des Körperteils relativ zu dem medizinischen Gerät (10) mindestens zwei Markierungen (22,24,26,28) aufweisen, welche eingerichtet sind, in genau definierten Positionen an dem Körperteil angebracht zu werden,

   (g) die bildsignalerzeugenden Sensoren (34,36) Mittel zur Erzeugung einer Pixelmatrix (50,52) enthalten, in welcher die Markierungen (22,24,26,28) als flächige Gebilde (54A,56A,58A,60A,54B,56B,58B,60B) erscheinen,

   (h) die bildverarbeitenden Mittel (74,76) zur Bestimmung der Schwerpunkte (80) der so erhaltenen flächigen Gebilde als Markierungspunkte ausgelegt sind, die durch die Markierungen (22,24,26,28) bestimmt sind und die Istposition des Körperteils definieren, und

   (i) die Signalverarbeitungs- und Reglermittel (90,92, 94) so zusammenwirken, daß die Ermittlung der Istposition des Körperteils aus der beobachteten optischen Informationen durch Berechnung erfolgt.

2. Vorrichtung nach Anspruch 1, **dadurch gekennzeichnet, daß** die Mittel (104;160,162,164) zur Verhinderung einer Behandlung bei Fehlausrichtung Stellgliedmittel umfaßt, die von den Stellsignalen beaufschlagt sind und durch welche bei einer Auswanderung des Körperteils aus der Sollposition die Ausrichtung zwischen Gerät (10) und Körperteil wiederherstellbar ist.

3. Vorrichtung nach Anspruch 2, **dadurch gekennzeichnet, daß** die Stellgliedmittel (104;160,162,164) zum Angreifen an dem Körperteil ausgebildet sind, so daß der Körperteil bei einer Auswanderung aus der Sollposition durch die Stellgliedmittel (104;160,162,164) in die Solposition zurückführbar ist.

4. Vorrichtung nach einem der Ansprüche 1 bis 3, **dadurch gekennzeichnet, daß** die Markierungen (22,24,26,28,30) von Kugeln gebildet sind, die in der Pixelmatrix (50,52) unabhängig von der Beobachtungsrichtung als kreisförmige Gebilde (54A,56A,58A,60A,54B,56B,58B,60B) erscheinen.

5. Vorrichtung nach Anspruch 4, **dadurch gekennzeichnet,** daß die Kugeln von kontrastierenden Flächen hinterlegt sind.

13

6. Vorrichtung nach Anspruch 4 oder 5, **dadurch gekennzeichnet, daß** eine Beleuchtungseinrichtung (38,40) zur Beleuchtung der Kugeln von mehreren Seiten vorgesehen ist.

7. Vorrichtung nach einem der Ansprüche 1 bis 6, **dadurch gekennzeichnet, daß** wenigstens drei Markierungen (22,24,26,28) vorgesehen sind.

8. Vorrichtung nach einem der Ansprüche 1 bis 7, zur genauen Positionierung des Kopfes eines Patienten relativ zu einer Bestrahlungseinrichtung (10), wobei der Behandlungspunkt in einem zubestrahlenden Tumor liegt, **gekennzeichnet durch**

   (a) einen Bestrahlungstisch (16) und einen auf dem Bestrahlungstisch in waagerechten Längsrichtung verschiebbaren Schlitten (15) zur Aufnahme des Patienten,

   (b) eine von dem Bestrahlungstisch (16) und Schlitten (15) gesonderte Kopfauflage (108), auf welcher bei Benutzung der Vorrichtung der Kopf des Patienten feststellbar aufliegt, wobei

   (c) ein erstes und ein zweites der besagten Stell-glieder (160,162)) in vertikaler Richtung und in waagerechter Querrichtung an der Kopfauflage (108) angreifen zur Bewegung derselben relativ zu dem Schlitten (15) und

   (d) ein drittes Stellglied (164) in der waagerechten Längsrichtung an dem Schlitten (15) angreift zur Längsverschiebung des Schlittens (15).

9. Vorrichtung nach Anspruch 8, **gekennzeichnet durch** weitere Stellglieder zur winkelmäßigen Ausrichtung des Körperteils.

10. Verfahren zur Positionierung des Kopfes eines Patienten zur Behandlung mit einem medizinischen Gerät (10), mit den Verfahrensschritten:

   (a) Anbringen eines mit Markierungen (22,24,26,28) versehenen und an das Gebiß des Patienten angepaßten Mundstückes an dem Patienten,

   (b) Vermessung der Markierungen (22,24,26,28) während einer ersten Behandlung, wenn der zubehandelnde, die Markierungen (22,24,26,28) tragende Kopf in einer vorgegebenen Sollage zu dem Gerät (10) fixiert ist, mittels wenigstens zweier bildsignalerzeugender Sensoren (36,38),

   (c) Bestimmung der Schwerpunkte (80) der von den Sensoren (36,38) erfaßten Abbilder (54A bis 62A; 54B bis 62B) der Markierungen (22,24,26,28) durch Bildverarbeitung in sensorfesten Koordinatensystemen,

   (d) Bestimmen der Lage der Schwerpunkte der Markierungen (22,24,26,28) in einem gerätefesten Koordinatensystem aus den in sensorfesten Koordinatensystemen bestimmten Schwerpunkten (80) der Abbilder (54A bis 62A; 54B bis 62B) als Maß für eine Sollage des Kopfes,

   (e) Vermessung der Markierungen (22,24,26,28) während einer nachfolgenden Behandlung mittels der gleichen, in gleicher Weise ausgerichteten Sensoren (36,38),

   (f) Bestimmen der Lageabweichung der Schwerpunkte der Markierungen (22,24,26,28) in dem gerätefesten Koordinatensystem aus den in den sensorfesten Koordinatensystemen bestimmten Schwerpunkten (80) der Abbilder (54A bis 62A; 54B bis 62B) und

   (g) Erzeugung von Stellsignalen nach Maßgabe der Lageabweichung und Korrektur der Lage des Kopfes nach Maßgabe der Stellsignale.

11. Verfahren nach Anspruch 10, **dadurch gekennzeichnet, daß** zur Kalibrierung der Abbildungseigenschaften der Optik der bildsignalerzeugenden Sensoren (36,38) ein mit Markierungen (146,148,150,152) versehener Testkörper (144) vermessen wird.

12. Verfahren nach Anspruch 11 oder 12, **dadurch gekennzeichnet, daß** zur Bestimmung der Lage (Position und Ausrichtung) der Sensoren (36,38) ein mit Markierungen (146,148,150,152) versehener Referenzkörper (144) vermessen wird, der eine wohldefinierte Lage in dem geratefesten Koordinatensystem hat.

**Claims**

1. A device for positioning a body part for treatment with a medical instrument (10) comprising

   (a) means (22, 24, 26, 28, 38, 40) for generating optical information regarding the position of the body part relative to the medical instrument (10),

   (b) at least two video signal generating sensors (34, 36) which are aligned to observe the optical information,

   (c) image processing means (74,76) for processing the video signals (54A, 56A, 58A, 60A, 54B, 56B, 58B, 60B) of the sensors (34,36) generated from the optical information,

   (d) signal processing means and control means (90, 92, 94)

   - for determining the actual position of the body part from the observed optical information,

   - for comparing the actual position with an ideal position, and

   - for generating actuating signals which depend on the control deviation between the actual position and the ideal position, and

   (e) means (104; 160, 162, 164) receiving the actuating signals for positioning the body part so that a precisely defined treatment point is held in an ideal position, and for preventing treatment when the body part and the instrument (10) are not properly aligned,
   characterized in that

   (f) the means (22, 24, 26, 28, 38, 40) for generating optical information regarding the position of the body part relative to the medical instrument (10) have at least two markings (22, 24, 26, 28) which are arranged to be applied in accurately defined positions to the body part,

   (g) the video signal generating sensors (34, 36) have means for generating a pixel matrix (50, 52) in which the markings (22, 24, 26, 28) appear as two-dimensional structures (54A, 56A, 58A, 60A, 54B, 56B, 58B, 60B),

   (h) the image processing means (74, 76) are designed for determining the centers of gravity (80) of the resulting two-dimensional structures as marking points which are determined by the markings (22, 24, 26, 28) and define the actual position of the body part, and

   (i) the signal processing means and control means (90, 92, 94) work together so that the actual position of the body part is determined from the observed optical information by performing calculations.

2. A device according to Claim 1, characterized in that the means (104; 160, 162, 164) for preventing a treatment when there is a faulty alignment comprises controlling element means which receive the actuating signals and restore the alignment between the instrument (10) and the body part if the body part moves out of the ideal position.

3. A device according to Claim 2, characterized in that the controlling element means (104; 160, 162, 164) are designed for gripping the body part, so that when the body part moves out of the ideal position, said body part can be returned to the ideal position by the controlling element means (104; 160, 162, 164)

4. A device according to one of Claims 1 through 3, characterized in that the markings (22, 24, 26, 28, 30) are formed by spheres which appear as circular structures (54A, 56A, 58A, 60A, 54B, 56B, 58B, 60B) in the pixel matrix (50, 52) regardless of the direction of observation.

5. A device according to Claim 4, characterized in that the spheres are backed by contrasting areas.

6. A device according to Claim 4 or 5, characterized in that a lighting device (38, 40) is provided for lighting the spheres from several sides.

7. A device according to one of Claims 1 through 6, characterized in that at least three markings (22, 24, 26, 28) are provided.

8. A device according to one of Claims 1 through 7, for accurate positioning a patient's head relative to a radiation device (10), with the treatment point being located in a tumor to be irradiated,
characterized by

(a) an irradiation table (16) and a carriage (15) to accommodate the patient displaceably in a horizontal longitudinal direction on the irradiation table,

(b) a head rest (108) which is separate from the irradiation table (16) and carriage (15) and on which the patient's head rests in a defined position when using the device, where

(c) a first and a second of said controlling elements (160, 162) grip the headrest (108) in the vertical direction and in the horizontal transverse direction in order to move said head rest relative to the carriage (15) and

(d) a third controlling element (164) grips the carriage (15) in the horizontal longitudinal direction for longitudinal displacement of the carriage (15).

9. A device according to Claim 8, characterized by additional controlling elements for angular orientation of the body part.

10. A method of positioning a patient's head for treatment with a medical instrument (10), comprising the process steps:

(a) securing on the patient a mouthpiece that has been provided with markings (22, 24, 26, 28) and is adapted to the patient's bite,

(b) measuring the markings (22, 24, 26, 28) by means of at least two video signal generating sensors (36, 38) during a first treatment when the head to be treated and having the markings (22, 24, 26, 28) is secured in a predetermined ideal position relative to the instrument (10),

(c) determining the centers of gravity (80) of the images (54A through 62A; 54B through 62B) of the markings (22, 24, 26, 28) detected by the sensors (36, 38) by image processing in sensor-fixed coordinate systems,

(d) determining the position of the centers of gravity of the markings (22, 24, 26, 28) in an instrument-fixed coordinate system from the centers of gravity (80) of the images (54A through 62A; 54B through 62B) determined in sensor-fixed coordinate systems as a measure of an ideal position of the head,

(e) measuring the markings (22, 24, 26, 28) during a subsequent treatment by means of the same sensors (36, 38) oriented in the same way,

(f) determining the deviation in position of the centers of gravity of the markings (22, 24, 26, 28) in the instrument-fixed coordinate system from the centers of gravity (80) of the images (54A through 62A; 54B through 62B) determined in the sensor-fixed coordinate systems, and

(g) generating actuating signals according to the deviation in position and correcting the position of the [patient's] head in accordance with the actuating signals.

11. A method according to Claim 10, characterized in that a test body (144) provided with markings (146, 148, 150, 152) is measured to calibrate the imaging properties of the optical lens of the video signal generating sensors (36, 38).

12. A method according to Claim 11 or 12, characterized in that a reference body (144) provided with markings (146, 148, 150, 152) and having a well-defined position in the instrument-fixed coordinate system is measured to determine the location (position and orientation) of the sensors (36, 38).

**Revendications**

1. Dispositif de positionnement d'une partie du corps en vue de son traitement avec un appareil médical (10), comprenant:

   (a) des moyens (22, 24, 26, 28, 38, 40) générant des informations optiques sur la position de la partie du corps par rapport à l'appareil médical (10),

   (b) au moins deux capteurs (34, 36) générant des signaux visuels, orientés en vue de l'observation des informations optiques,

   (c) des moyens de traitement d'images (74, 76) traitant les signaux visuels (54A, 56A, 58A, 60A, 54B, 56B, 58B, 60B) des capteurs (34, 36), générés au départ des informations optiques,

   (d) des moyens de traitement et de régulation des signaux (90, 92, 94)

   - pour déterminer la position réelle de la partie du corps à partir des informations optiques observées,

   - pour comparer la position réelle à une position de consigne et

   - pour générer des signaux de réglage qui sont tributaires de l'écart-type entre la position réelle et la position de consigne, et

   (e) des moyens (104; 160, 162, 164) qui sont sous l'influence des signaux de réglage, pour positionner la partie du corps, de façon à ce qu'un point de traitement rigoureusement défini soit maintenu dans une position de consigne, et pour éviter un traitement si l'orientation de l'appareil (10) par rapport à la partie du corps est mauvaise,
   caractérisé en ce que:

   (f) les moyens (22, 24, 26, 28, 38, 40) générant des informations optiques sur la position de la partie du corps par rapport à l'appareil médical (10) comportent au moins deux repères (22, 24, 26, 28) qui sont adaptés pour être placés en des emplacements définis sur la partie du corps,

   (g) les capteurs (34, 36) générant des signaux visuels comprennent des moyens générant une matrice de pixels (50, 52) dans laquelle les repères (22, 24, 26, 28) apparaissent sous forme de structures bidimensionnelles (54A, 56A, 58A, 60A, 54B, 56B, 58B, 60B),

   (h) les moyens de traitement d'images (74, 76) déterminant les barycentres (80) des structures bidimensionnelles sont conçus sous forme de points de repère qui sont déterminés par les repères (22, 24, 26, 28) et définissent la position réelle de la partie du corps, et

   (i) les moyens de traitement et de régulation de signaux (90, 92, 94) coopèrent de telle sorte que la détermination de la position réelle de la partie du corps à partir des informations optiques observées se fait par calcul.

2. Dispositif selon la revendication 1, caractérisé en ce que les moyens (104; 160, 162, 164) empêchant un traitement en cas de mauvaise orientation comprennent des servo-positionneurs qui sont sous l'influence des signaux de réglage et permettent un rétablissement de l'orientation de l'appareil (10) par rapport à la partie du corps si la partie du corps s'écarte de la position de consigne.

3. Dispositif selon la revendication 2, caractérisé en ce que les servo-positionneurs (104; 160, 162, 164) sont adaptés pour intervenir sur la partie du corps de telle façon que dans le cas d'un écartement par rapport à la position de consigne, la partie du corps puisse être replacée dans la position de consigne par les servo-positionneurs (104; 160, 162, 164)

4. Dispositif selon l'une quelconque des revendications 1 à 3, caractérisé en ce que les repères (22, 24, 26, 28, 30) sont constitués par des sphères qui apparaissent dans la matrice de pixels (50, 52), indépendamment de la direction d'observation, sous forme de structures circulaires (54A, 56A, 58A, 60A, 54B, 56B, 58B, 60B).

5. Dispositif selon la revendication 4, caractérisé en ce que les sphères sont doublées de surfaces contrastees.

6. Dispositif selon la revendication 4 ou 5, caractérisé en ce qu'un dispositif d'éclairage (38, 40) a été prévu afin d'éclairer les sphères de plusieurs côtés.

7. Dispositif selon l'une quelconque des revendications 1 à 6, caractérisé en ce qu'au moins trois repères (22, 24, 26, 28) ont été prévus.

8. Dispositif selon l'une quelconque des revendications 1 à 7, pour le positionnement précis de la tête d'un patient par rapport à un dispositif d'irradiation (10), le point de traitement se trouvant dans une tumeur qu'il s'agit d'irradier, caractérisé par:

(a) une table d'irradiation (16) et un chariot (15) coulissant dans le sens longitudinal horizontal sur la table d'irradiation, destiné à accueillir le patient,

(b) un support pour tête (108) séparé de la table d'irradiation (16) et du chariot (15), et sur lequel la tête du patient peut être calée lors de l'utilisation du dispositif,
dispositif dans lequel

(c) un premier et un deuxième desdits servo-positionneurs (160, 162) interviennent dans la direction verticale et dans la direction transversale horizontale sur le support pour tête (108), pour le faire bouger par rapport au chariot (15) et

(d) un troisième servo-positionneur (164) intervient sur le chariot (15) dans la direction longitudinale horizontale, pour faire glisser le chariot (15) longitudinalement.

9. Dispositif selon la revendication 8, caractérisé par d'autres servo-positionneurs pour l'orientation angulaire de la partie du corps.

10. Procédé de positionnement de la tête d'un patient en vue de son traitement avec un appareil médical (10), comprenant les opérations consistant à:

(a) poser sur le patient un élément buccal muni de repères (22, 24, 26, 28) et adapté à la dentition du patient,

(b) mesurer les repères (22, 24, 26, 28) pendant un premier traitement, lorsque la tête à traiter portant les repères (22, 24, 26, 28) est calée dans une position de consigne prédéterminée par rapport à l'appareil (10), au moyen d'au moins deux capteurs (36, 38) générant des signaux visuels.

(c) déterminer les barycentres (80) des images (54A à 62A; 54B à 62B) des repères (22, 24, 26, 28) détectées par les capteurs (36, 38), par un traitement d'images dans des systèmes de coordonnées implantés dans les capteurs,

(d) déterminer la position des barycentres des repères (22, 24, 26, 28) dans un système de coordonnées implanté dans l'appareil, à partir des barycentres (80) des images (54A à 62A; 54B à 62B) déterminés dans les systèmes de coordonnées implantés dans les capteurs, en tant que mesure pour une position de consigne de la tête,

(e) mesurer les repères (22, 24, 26, 28) pendant un traitement subséquent, au moyen des mêmes capteurs (36, 38) orientés de la même manière,

(f) déterminer l'écart de position des barycentres des repères (22, 24, 26, 28) dans le système de coordonnées implanté dans l'appareil à partir des barycentres (80) des images (54A à 62A; 54B à 62B) déterminés dans les systèmes de coordonnées implantés dans les capteurs, et

(g) générer des signaux de réglage en fonction de l'écart de position et corriger la position de la tête en fonction des signaux de réglage.

11. Procédé selon la revendication 10, caractérisé en ce que pour calibrer les caractéristiques de reproduction de l'optique des capteurs générant des signaux visuels (36, 38), on mesure une éprouvette (144) munie de repères (146, 148, 150, 152).

12. Procédé selon la revendication 10 ou 11, caractérisé en ce que pour déterminer l'assiette (position et orientation) des capteurs (36, 38), on mesure un corps de référence (144) muni de repères (146, 148, 150, 152), qui occupe une position bien déterminée dans le système de coordonnées implanté dans l'appareil.

Fig.1

Fig. 10

Fig. 2

Fig. 3

Fig. 9

```
┌─────────────────┐              ┌─────────────────┐
│    SENSOR 1     │ 34           │    SENSOR 2     │ 36
└─────────────────┘              └─────────────────┘

┌─────────────────┐ 50           ┌─────────────────┐ 52
│   PIXELMATRIX   │              │   PIXELMATRIX   │
└─────────────────┘              └─────────────────┘

┌─────────────────┐ 74           ┌─────────────────┐ 76
│  SCHWERPUNKT-   │              │  SCHWERPUNKT-   │
│ BILDUNG DER AB- │              │ BILDUNG DER AB- │
│  GEBILDETEN     │              │  GEBILDETEN     │
│  MARKEN         │              │  MARKEN         │
└─────────────────┘              └─────────────────┘

         ┌──────────────────────────────┐ 90
         │ LAGEBESTIMMUNG               │
         │ DES KÖRPERTEILS              │
         └──────────────────────────────┘

  92
┌─────────────────┐    ┌─────────────────┐ 94
│   SOLLWERTE     │────│    REGLER       │
└─────────────────┘    └─────────────────┘
                              │  98

                       ┌─────────────────┐ 104
                       │     STELL-      │
                       │    GLIEDER      │
                       └─────────────────┘
```

Fig. 4

22

Fig. 5

EP 0 560 331 B1

Fig. 6

Fig. 7

Fig. 8